(19) Europäisches Patentamt
European Patent Office
Office européen des brevets

(11) Publication number : **0 348 121 B1**

# (12) EUROPEAN PATENT SPECIFICATION

(45) Date of publication of patent specification : **07.04.93 Bulletin 93/14**

(51) Int. Cl.[5] : **C07D 311/62,** A61K 31/35, A61K 7/26, C07C 47/565

(21) Application number : **89306163.0**

(22) Date of filing : **19.06.89**

## (54) Process for the synthesis of anthocyanidines and novel intermediates produced in said processes.

(30) Priority : **20.06.88 GB 8814603**

(43) Date of publication of application : **27.12.89 Bulletin 89/52**

(45) Publication of the grant of the patent : **07.04.93 Bulletin 93/14**

(84) Designated Contracting States : **AT BE CH DE ES FR GB GR IT LI LU NL SE**

(56) References cited :
**GB-A- 1 595 351**
**H.R. Christen "Grundlagen der Organischen Chemie" (1972), ISBN 3-1941-0043-3, pages 581 and 582**

(73) Proprietor : **IdB Holding S.p.A.**
**Via Ripamonti, 99**
**I-20141 Milano (IT)**

(72) Inventor : **Bruno, Gabetta**
**Via Verona, 13**
**Milano (IT)**
Inventor : **Raffaello, Giorgi**
**Via Monzambano, 13**
**Milano (IT)**
Inventor : **Giorgio, Pifferi**
**Via C.A. Carlone, 8**
**Milano (IT)**

(74) Representative : **Ritter, Stephen David et al**
**Mathys & Squire 10 Fleet Street**
**London EC4Y 1AY (GB)**

EP 0 348 121 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a novel process for the synthesis of anthocyanidines and to a novel intermediate produced in said process.

Anthocyanidines are polyphenols which are widely distributed in nature in the form of glucosides known as anthocyanins. Anthocyanins are substances which are largely responsible for the colouring of flowers and fruits.

As used herein the term "anthocyanidine" refers to a substituted 2-arylbenzopyran derivative of the formula

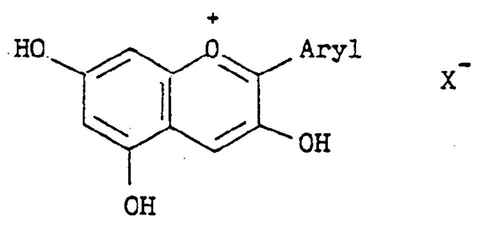

wherein $X^-$ represents an anion and "Aryl" represents an aryl group, particularly a phenyl group substituted by one or more hydroxyl groups or lower alkoxy groups. As used herein, the term "lower" as applied e.g. to an alkyl or alkoxy group means such a group containing 1 to 6 carbon atoms.

The most common anthocyanidines are represented by formula

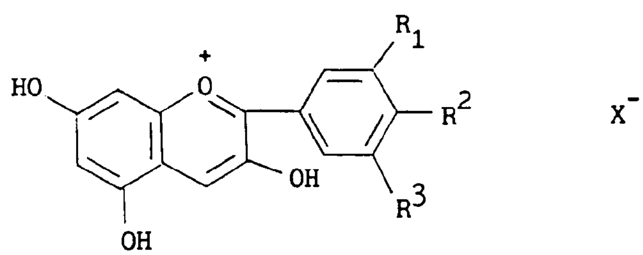

in which

a $R_1$=$R_3$=H, $R_2$=OH
b $R_1$=$R_2$=OH, $R_3$=H
c $R_1$=$R_2$=$R_3$=OH
d $R_1$=OMe, $R_2$=OH, $R_3$=H
e $R_1$=$R_2$=OH, $R_3$=OMe
f $R_1$=$R_3$=OMe, $R_2$=OH

and $X^-$ is an anion, e.g. $Cl^-$.

The anthocyanidines which are most widespread in nature (Pelargonidine, Ia; Cyanidine, Ib; Delphinidine, Ic; Peonidine, Id; Petunidine, Ie; Malvidine, If) differ from one another in the nature of the substituent or substituents attached to the benzene ring in the 2 position of the 2-arylbenzopyran nucleus. Anthocyanidines are endowed with remarkable healing properties and are therefore particularly useful in the treatment of skin injuries, torpid wounds and internal and external ulcers. They moreover possess antiinflammatory, vasoprotective, hypolipemic, hypocholesteremic and hypoglycemic activity. As many anthocyanidines are characterised by low toxicity, they are particularly useful for prolonged therapeutic treatments (see GB-1589294, GB-1595351; Inverni Della Beffa SpA).

One method of preparing anthocyanidines is by acid hydrolysis of naturally occurring anthocyanins. In addition to preparation by acid hydrolysis of anthocyanins, known methods of preparing anthocyanidines include semi-synthesis from natural substances such as flavones and catechols which already contain the 2-phenylbenzopyran nucleus, or total synthesis. One such total synthesis is that effected by R. Robinson (The Chemistry of Flavonoid Compounds, Pergamon Press, Oxford, 1962; J.B. Harborne, T.J. Mabry, H. Mabry, The Flavonoids, Chapman and Hall, London, 1975), illustrated in Scheme 1 below.

This procedure involves the conversion of phloroglucinol (II) into phloroglucinaldehyde (III), the selective O-benzoylation of the latter to the 2-O-benzoate (IV′), the condensation of (IV′) with the appropriate acetophenone derivative (V') to give the protected anthocyanidine (VI') which, following basic hydrolysis, produces an anthocyanidine, e.g. (I a-f).

Scheme 1

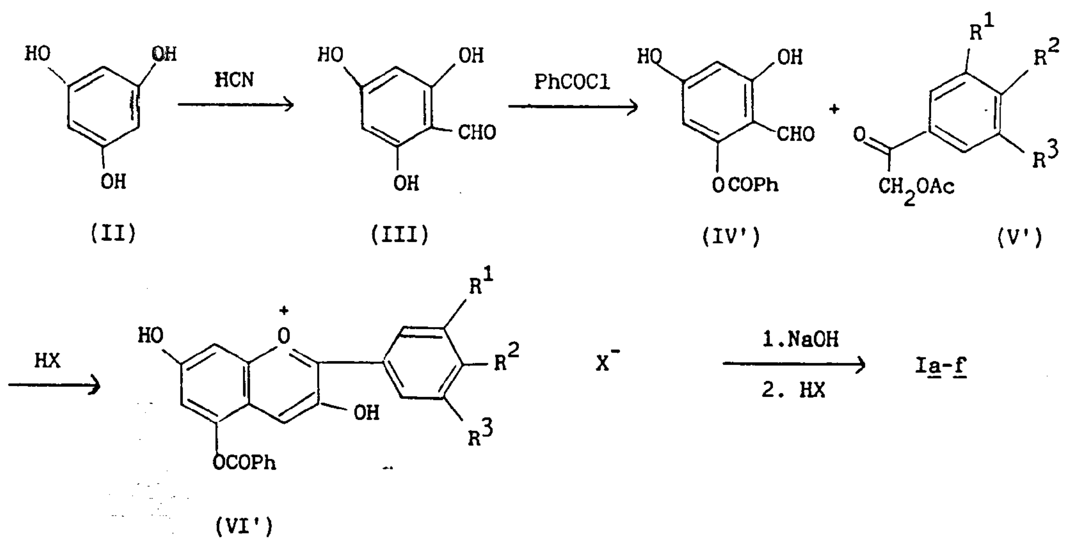


(II) (III) (IV') (V') (VI')

Various disadvantages are associated with each of the above-mentioned procedures for the preparation of anthocyanidines. All are difficult to effect on an industrial scale. Semi-synthesis from natural flavonic synthons takes place with low yields and leads to impure final products. Also the starting materials (e.g. anthocyanins, flavones and catechols) are not in plentiful supply. The procedures involving total synthesis are also unsatisfactory. Thus the yields are also low (4-6%) in Robinson's synthesis and dangerous reagents such as hydrocyanic acid are employed.

Particularly, disadvantages are encountered in Robinson's synthesis in the preparation of phloroglucinaldehyde (III). Three synthetic methods are described. A first provides for the reaction of phloroglucinol (II) with hydrocyanic acid in the presence of acid catalysts (D. Pratt et al., J. Chem. Soc. 1132, 1925). A second requires reaction of phloroglucinol with dichloromethyl thioethers in the presence of titanium tetrachloride (H. Gross et al., Ber. 97, 2606, 1964) and in the third, phloroglucinol is reacted with alkyl orthoformates in the presence of aluminium chloride (H. Gross et al., Ber. 96, 308, 1963). All these procedures are laborious, lead to phloroglucinaldehyde of unsatisfactory quality and purity and are further characterised by low yields. Accordingly there is a need for an improved process of producing phloroglucinaldehyde with high yield and purity.

It is also to be noted that in Robinson's procedure, phloroglucinaldehyde (III) cannot be utilized as such for the construction of the anthocyanidine nucleus since, at the stage when it is condensed with the acetophenone derivative (V'), it can give rise to polymerisation products. In order to avoid this, Robinson proposes the use of the 2-O-benzoate (IV') (R. Robinson et al., J. Chem. Soc. 2672, 1931), which can be obtained from phloroglucinaldehyde (III) by selective benzoylation. However, the procedure proposed by Robinson produces a yield of only 35% with phloroglucinaldehyde 2,4-di-O-benzoate and phloroglucinaldehyde 2,4,6-tri-O-benzoate present as impurities.

The impurities create a problem in that the subsequent synthesis process requires the use of intermediates which are free from impurities in order to avoid the formation of by-products which cannot be eliminated from the final anthocyanidines by normal methods of purification. Thus there is a further need for a process which can provide a suitable intermediate in relatively pure form.

The present invention overcomes the problems outlined above by providing a new process for the synthesis of anthocyanidines and a novel intermediate for use therein. The novel process for producing anthocyanidines which involve the use of a 2,4-diprotected phloroglucinaldehyde derivative as intermediate.

Thus according to one aspect of the present invention there is provided a process of producing an anthocyanidine which comprises

(a) reacting phloroglucinaldehyde with a reagent capable of introducing benzoyl groups so as to form a 2,4,6-tri-O-protected derivative of phloroglucinaldehyde of formula

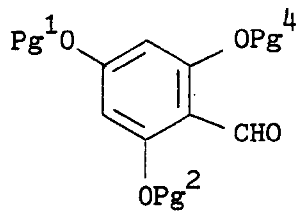

VIII

wherein $Pg^1$, $Pg^2$ and $Pg^4$ represent benzoyl groups,

(b) selectively removing the benzoyl group $Pg^4$ at position 6 so as to produce a 2,4-di-O-protected derivative of phloroglucinaldehyde of formula VII

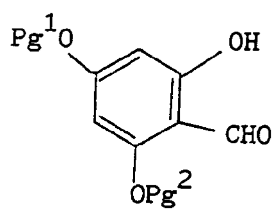

VII

wherein $Pg^1$ and $Pg^2$ each represents a benzoyl group,

(c) reacting the so-formed compound of formula VII with a compound of formula

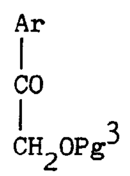

wherein Ar represents optionally substituted aryl and $Pg^3$ represents a carboxylic acid acyl group, to form a compound of formula

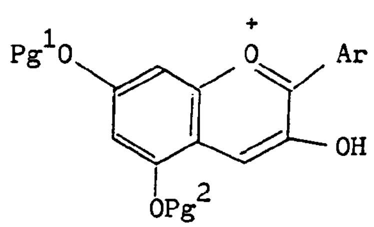

$X^-$ IX

wherein $Pg^1$ and $Pg^2$ and Ar are as defined above and $X^-$ is an anion, and

(d) converting protected hydroxyl groups to free hydroxyl groups.

As indicated above, a key feature of the overall process of the invention is the use of 2,4-di-O-benzoyl phloroglucinaldehyde derivative as an intermediate. The production of this intermediate comprises reacting phloroglucinaldehyde with a reagent capable of introducing benzoyl groups so as to form a 2,4,6-tri-O-benzoyl derivative of phloroglucinaldehyde of formula

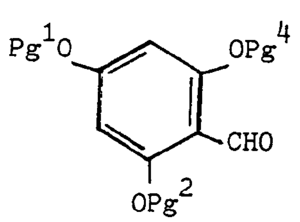

VIII

wherein $Pg^1$, $Pg^2$ and $Pg^4$ represents benzoyl groups and selectively removing the benzoyl group $Pg^4$ at pos-

ition 6 so as to yield the desired compound of formula VII.

In this procedure the reagent used to produce the intermediate of formula VIII is preferably benzoic acid or an ester forming derivative thereof, e.g. benzoyl chloride.

The benzoyl group at position 6 may be removed by subjecting the intermediate of formula VIII to selective hydrolysis under mild conditions, e.g. in the presence of water and a weak base such as triethylamine to give VII.

The phloroglucinaldehyde of formula

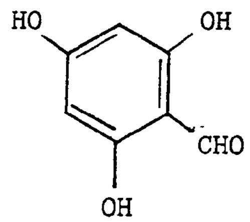

III

may be produced by hydrolysing a compound of formula

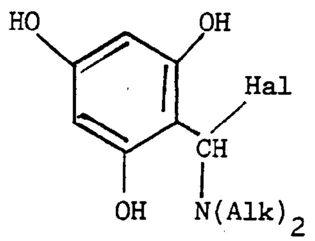

X

wherein Hal represents a halogen atom and each Alk represents a lower alkyl group.

In this procedure, Hal is preferably chlorine and Alk is preferably methyl. The compound of formula X is preferably prepared by reacting phloroglucinol (II) with a formamide of formula

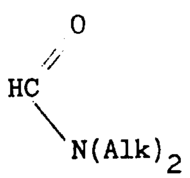

XII

wherein Alk is as defined above, in the presence of a phosphorus oxyhalide.

By way of specific example, the overall process of the invention may be represented by the following scheme.

Scheme 2

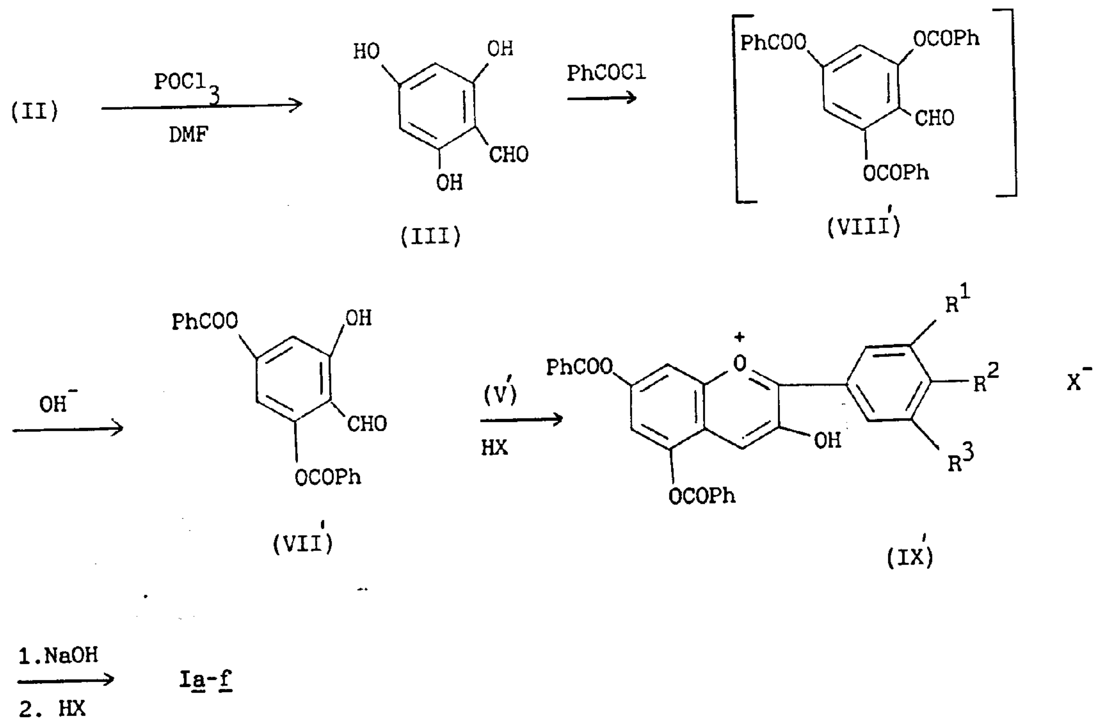


In this Scheme, preferred protecting groups and reagents are shown. It is to be appreciated however that alternative protecting groups and reagents may be employed, as described above.

Referring to the above Scheme, we have found that intermediate II can be easily formylated by treatment with dimethylformamide and phosphorus oxychloride in an aprotic solvent. The adduct formed is collected by filtration and hydrolysed by heating in water, from which, after cooling, the phloroglucinaldehyde III crystallizes with high yield and purity.

Moreover we have found that significant advantages ensue from the use of the 2,4-dibenzoate VII′ for reaction with acetophenone derivative V′, instead of the monobenzoate used in known analogous procedures. Thus as shown in the above Scheme 2, there are obtained the 5,7-dibenzoylanthocyanidines IX′ (which are novel compounds), from which anthocyanidines can be readily obtained in high yields by means of alkaline hydrolysis followed by acidification.

We have found that the dibenzoate VII′ cannot be prepared in satisfactory yields by direct benzoylation of the phloroglucinaldehyde (III), as mixtures of mono-, di- and tribenzoates are obtained. The present invention however provides an efficient method of synthesis of dibenzoate VII′ which involves the perbenzoylation of III with benzoyl chloride, e.g. in solution in acetone and in the presence of a tertiary organic base, followed by selective hydrolysis of VIII′, e.g. in aqueous triethylamine at 0-30°C. With this new method, the conversion of III into VII′ is practically quantitative.

In the specific example outlined above the present invention is therefore characterised by a novel procedure of preparation of phloroglucinaldehyde (III) from phloroglucinol (II) and by a novel procedure of preparation of phloroglucinaldehyde 2,4-di-O-benzoate (VII′) from the corresponding tri-O-benzoate (VIII′), obtainable from phloroglucinaldehyde (III) by exhaustive benzoylation.

Moreover, the convenience of the use of the intermediate VII′ in the synthesis of the 2-arylbenzopyran ring and of the new dibenzoates IX′ for the preparation of the anthocyanidines I offer further advantages.

The new processes are easily scaled up for use on an industrial scale and avoid the use of highly toxic reagents. Other advantages reside in a distinct improvement in the total yield and in the possibility of obtaining anthocyanidines with a high degree of purity.

The processes according to the invention will be illustrated in the following examples.

## Example 1 - Synthesis of Phloroglucinaldehyde (III)

9 l (1.47 kg, 96.4 moles) of phosphorus oxychloride are added to a solution containing 11.2 kg (88.9 moles) of anhydrous phloroglucinol (II) and 7.5 l (7 kg, 96.4 moles) of N,N-dimethylformamide in 33 l of ethyl acetate. The precipitated solid is filtered and the residue is dissolved in hot water. After cooling to room temperature, the crystallizate is filtered. 11.4 kg of III are obtained (yield: 83%) having a m.p. of 185°-186°C, $E^{1\%}_{1cm}$1265 at 292 nm ($CH_3OH$), IR bands (KBr) at 3300, 1635 and 1600 $cm^{-1}$, $M^+$ at m/z 154.

## Example 2 - Synthesis of Phloroglucinaldehyde 2,4-di-O-benzoate (VII′) After Isolation of (VIII′)

12.5 l (9.12 kg, 91.5 moles) of triethylamine are added under agitation to a solution containing 4.7 kg (30.5 moles) of phloroglucinaldehyde (III) and 10.6 l (12.8 kg, 91.5 moles) of benzoyl chloride in 45 l of acetone. The precipitate is removed by centrifuging and the organic phase is concentrated. By dilution with deionized water, 14.2 kg of VIII′ are obtained in essentially quantitative yield; m.p. 111°-112°C, $E^{1\%}_{1cm}$970 at 235 nm ($CH_3OH$), $M^+$ at m/z 466.

The solid obtained is dissolved in 85 l of acetone and 1.6 l of water and 1.1 l of triethylamine are added. After standing overnight at room temperature, the reaction mixture is concentrated and poured into a dilute aqueous solution of sodium bicarbonate. The precipitated solid (10.5 kg) of VII′ (yield: 95%) is collected by filtration; m.p. 122°-123°C, $E^{1\%}_{1cm}$526 at 268 nm (dioxan), $M^+$ at m/z 362.

## Example 3 - Direct Synthesis of Phloroglucinaldehyde 2,4-di-O-benzoate (VII′)

A solution containing 4.7 kg (30.5 moles) of III in 45 l of acetone is treated with 10.6 l (12.8 kg, 91.5 moles) of benzoyl chloride in the presence of 12.5 l (9.12 kg, 91.5 moles) of triethylamine by the procedure described in Example 2. After the filtration of the solid which is separated, the acetone solution is diluted with 1.1 l of triethylamine and 1.6 l of water, allowed to stand over night, concentrated and poured into a dilute aqueous solution of sodium bicarbonate. The precipitated solid is filtered and is constituted by 10.5 kg of VII′ (yield: 95%), identical (mixed melting point and extinction coefficient) to that described in Example 2.

## Example 4 - Synthesis of Pelargonidine Chloride (Ia, X=$Cl^-$)

400 g of hydrogen chloride gas are added under agitation to a suspension containing 500 g (1.38 moles) of phloroglucinaldehyde 2,4-di-O-benzoate (VII) and 362 g (1.53 moles) of omega-acetoxy-4-acetoxyacetophenone (Va) in 4 l of a 4:1 ethyl acetate-ethanol mixture and the suspension is then left to stand overnight at room temperature. The precipitated solid is collected by filtration and is constituted by 580 g of pelargonidine chloride 5,7-di-O-benzoate (IXa) (1.13 moles; yield: 81%), which is treated with 4 l of a 50% water-methanol solution containing 130 g of sodium hydroxide and then with 4 l of 25% hydrochloric acid. The precipitated solid is filtered and crystallized from a 3:1 methanol concentrated hydrochloric acid mixture. 285 g of pelargonidine chloride are obtained in the form of monohydrate (yield: 78%), m.p. 302°C (dec.), $E^{1\%}_{1cm}$1131 at 525 nm (MeOH), HPLC assay 99.2%.

## Example 5 - Synthesis of Cyanidine Chloride (Ib, X=$Cl^-$)

1.5 kg of hydrogen chloride gas are added under agitation to a suspension containing 1.5 kg (4.14 moles) of phloroglucinaldehyde, 2,4-di-O-benzoate (VII) and 1.35 kg (4.59 moles) of omega-acetoxy-3, 4-diacetoxyacetophenone in 15 l of a 4:1 ethyl acetate-ethanol mixture and the suspension is then left to stand overnight at room temperature. The separated solid, constituted by 1.75 kg of cyanidine chloride, 5,7-di-O-benzoate (IXb) (3.29 moles, yield: 79%) is collected by filtration and treated with 15 l of a 50% water-methanol solution containing 500 g of sodium hydroxide and then with 15 l of 25% hydrochloric acid. The precipitated solid is filtered and crystallized from a 3:1 methanol-concentrated hydrochloric acid mixture. 1.02 kg (2.93 moles) of cyanidine chloride are obtained in the form of monohydrate (yield: 89%, m.p. 300° (dec.), $E^{1\%}_{1cm}$1220 at 540 nm (MeOH), HPLC assay 98.9%.

## Claims

1. A process of producing an anthocyanidine which comprises
   (a) reacting phloroglucinaldehyde with a reagent capable of introducing benzoyl groups so as to form a 2,4,6-tri-O-protected derivative of phloroglucinaldehyde of formula

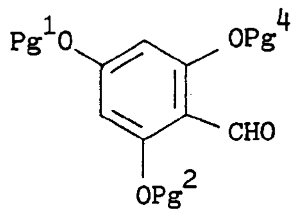

VIII

   wherein $Pg^1$, $Pg^2$ and $Pg^4$ represent benzoyl groups,
   (b) selectively removing the benzoyl group $Pg^4$ at position 6 so as to produce a 2,4-di-O-protected derivative of phloroglucinaldehyde of formula VII

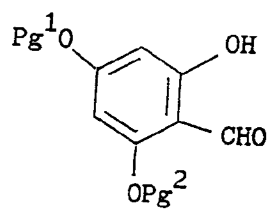

VII

   wherein $Pg^1$ and $Pg^2$ each represents a benzoyl group,
   (c) reacting the so-formed compound of formula VII with a compound of formula

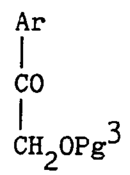

   wherein Ar represents optionally substituted aryl and $Pg^3$ represents a carboxylic acid acyl group, to form a compound of formula

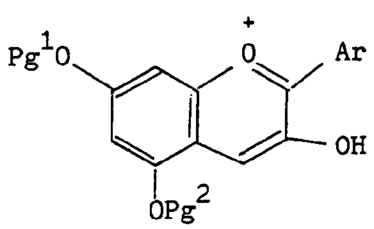

$X^-$ IX

   wherein $Pg^1$ and $Pg^2$ and Ar are as defined above and $X^-$ is an anion, and
   (d) converting protected hydroxyl groups to free hydroxyl groups.

2. A process according to Claim 1 wherein said reagent is benzoic acid or an ester-forming derivative thereof.

3. A process according to Claim 2 wherein said reagent is benzoyl chloride.

4. A process according to any preceding claim wherein the benzoyl group $Pg^4$ at position 6 is selectively removed by subjecting the compound of formula VIII to hydrolysis under mild conditions.

5. A process according to Claim 4 wherein the hydrolysis is carried out in the presence of water and a weak base.

6. A process according to Claim 5 wherein the weak base is triethylamine.

7. A process according to any of Claims 4 to 6 wherein the hydrolysis is carried out at a temperature of from 0°C to 30°C.

8. A process according to any preceding claim wherein the formed anthocyanidine has the formula

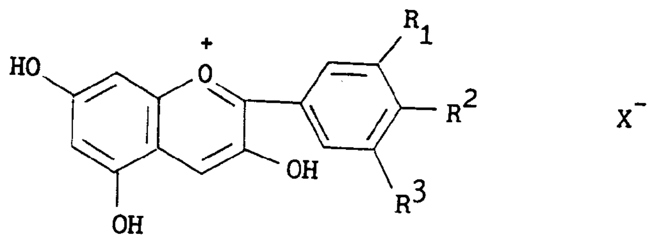

wherein $R_1$, $R_2$ and $R_3$ are independently selected from hydrogen, hydroxyl and methoxyl groups and $X^-$ is an anion.

9. A process according to Claim 8 wherein $R_1$, $R_2$ and $R_3$ have one of the following meanings:
a $R_1$=$R_3$=H, $R_2$=OH
b $R_1$=$R_2$=OH, $R_3$=H
c $R_1$=$R_2$=$R_3$=OH
d $R_1$=OMe, $R_2$=OH, $R_3$=H
e $R_1$=$R_2$=OH, $R_3$=OMe
f $R_1$=$R_3$=OMe, $R_2$=OH

10. The compound 2,4-di-O-benzoyl phloroglucinaldehyde having the formula

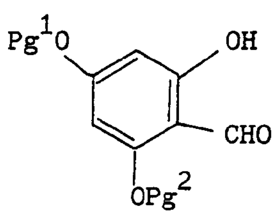

VII

in substantially pure form wherein $Pg^1$ and $Pg^2$ each represents benzoyl.

11. The use of 2,4-di-O-benzoyl phloroglucinaldehyde of formula

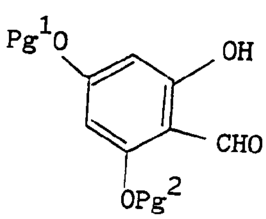

VII

wherein $Pg^1$ and $Pg^2$ are each benzoyl, as an intermediate for the synthesis of anthocyanidines.

**Patentansprüche**

1. Verfahren zur Herstellung eines Anthocyanidins, bei dem man:

   (a) Phloroglucinaldehyd mit einem Reagenz, das in der Lage ist, Benzoylgruppen einzuführen, um so ein 2,4,6-Tri-O-geschütztes Phloroglucinaldehydderivat herzustellen, umsetzt, wobei das Phloroglucinaldehydderivat folgende Formel aufweist:

   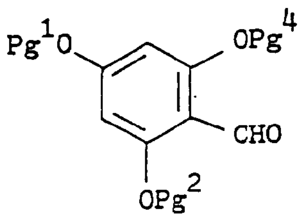

   VIII ,

   in der $Pg^1$, $Pg^2$ und $Pg^4$ Benzoylgruppen bedeuten,

   (b) die Benzoylgruppe $Pg^4$ in Stellung 6 selektiv entfernt, um auf diese Weise ein 2,4-Di-O-geschütztes Phloroglucinaldehydderivat der Formel VII herzustellen

   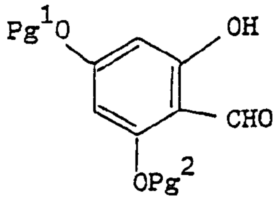

   VII ,

   in der $Pg^1$ und $Pg^2$ jeweils eine Benzoylgruppe bedeuten,

   (c) die so gebildete Verbindung der Formel VII mit einer Verbindung der Formel:

   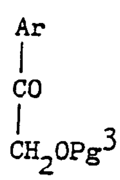

   umsetzt, in der Ar ein gegebenenfalls substituiertes Aryl bedeutet und $Pg^3$ eine Carbonsäureacylgruppe bedeutet, um eine Verbindung der Formel

   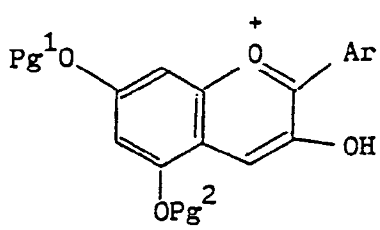

   $X^-$ IX

   herzustellen, in der $Pg^1$ und $Pg^2$ und Ar die vorstehend gegebene Bedeutung aufweisen und $X^-$ ein Anion ist, und

   (d) die geschützten Hydroxylgruppen in freie Hydroxylgruppen überführt.

2. Verfahren nach Anspruch 1, bei dem das Reagenz Benzoesäure oder einer esterbildendes Derivat davon ist.

3. Verfahren nach Anspruch 2, bei dem das Reagenz Benzoylchlorid ist.

4. Verfahren nach einem der vorhergehenden Ansprüche, bei dem die Benzoylgruppe $Pg^4$ in Stellung 6 selektiv entfernt wird, indem man die Verbindung der Formel VIII einer Hydrolyse unter milden Bedingungen unterwirft.

5. Verfahren nach Anspruch 4, bei dem die Hydrolyse in Gegenwart von Wasser und einer schwachen Base durchgeführt wird.

6. Verfahren nach Anspruch 5, bei dem die schwache Base Triethylamin ist.

7. Verfahren nach einem der Ansprüche 4 bis 6, bei dem die Hydrolyse bei einer Temperatur von 0°C bis 30°C durchgeführt wird.

8. Verfahren nach einem der vorhergehenden Ansprüche, bei dem das gebildete Anthocyanidin die folgende Formel aufweist

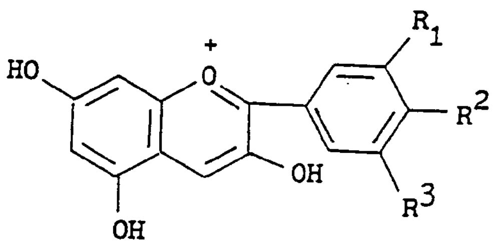

$X^-$ ,

in der $R_1$, $R_2$ und $R_3$ unabhängig aus Wasserstoff, Hydroxyl und Methoxygruppen ausgewählt sind und $X^-$ ein Anion ist.

9. Verfahren nach Anspruch 8, bei dem $R_1$, $R_2$ und $R_3$ eine der folgenden Bedeutungen aufweisen:
   - a $R_1$=$R_3$=H, $R_2$=OH
   - b $R_1$=$R_2$=OH, $R_3$=H
   - c $R_1$=$R_2$=$R_3$=OH
   - d $R_1$=OMe, $R_2$=OH, $R_3$=H
   - e $R_1$=$R_2$=OH, $R_3$=OMe
   - f $R_1$=$R_3$=OMe, $R_2$=OH

10. 2,4-Di-O-benzoyl-phloroglucinaldehyd der Formel:

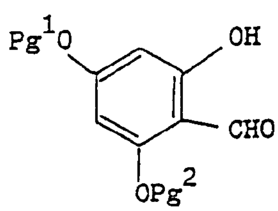

VII,

in im wesentlichen reiner Form, in der $Pg^1$ und $Pg^2$ jeweils Benzoyl bedeuten.

11. Verwendung von 2,4-Di-O-benzoyl-phloroglucinaldehyd der Formel:

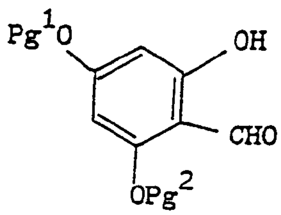

VII,

in der $Pg^1$ und $Pg^2$ jeweils Benzoyl bedeuten, als Zwischenprodukt für die Synthese von Anthocyanidinen.

## Revendications

1. Procédé pour la production d'une anthocyanidine qui comprend :
   (a) la réaction du phloroglucinaldéhyde avec un réactif capable de produire des groupes benzoyle de façon à former un dérivé 2,4,6-tri-O-protégé du phloroglucinaldéhyde de formule VIII:

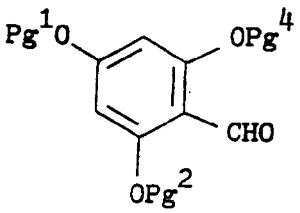

VIII

   dans laquelle $Pg^1$, $Pg^2$ et $Pg^4$ représentent des groupes benzoyles,
   (b) l'élimination sélective du groupe benzoyle $Pg^4$ en position 6 de façon à former un dérivé 2,4-di-O-protégé du phloroglucinaldéhyde de formule VII:

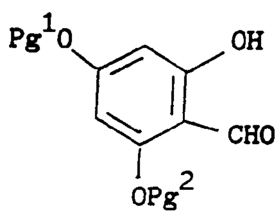

VII

   dans laquelle $Pg^1$ et $Pg^2$ représentent chacun un groupe benzoyle,
   (c) la réaction du composé ainsi formé de formule VII avec un composé de formule :

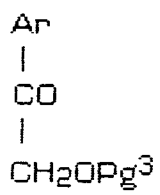

   dans laquelle Ar est un groupe aryle éventuellement substitué et $Pg^3$ est un groupe acyle d'acide carboxylique, pour former un groupe de formule IX :

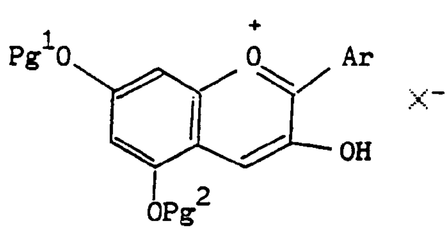

IX

   dans laquelle $Pg^1$ et $Pg^2$ et Ar sont tels que définis plus haut et $X^-$ est un anion, et
   (d) la conversion des groupes hydroxy protégés en groupes hydroxy libres.

2. Procédé suivant la revendication 1, dans lequel ce réactif est l'acide benzoïque ou un dérivé de celui-ci formant un ester.

3. Procédé suivant la revendication 2, dans lequel ce réactif est le chlorure de benzoyle.

4. Procédé suivant l'une quelconque des revendications précédentes, dans lequel le groupe benzoyle $Pg^4$

en position 6 est éliminé sélectivement par soumission du composé de formule VIII à une hydrolyse dans des conditions douces.

5. Procécé suivant la revendication 4, dans lequel l'hydrolyse est conduite en présence d'eau et d'une base faible.

6. Procédé suivant la revendication 5, dans lequel la base faible est la triéthylamine.

7. Procédé suivant l'une quelconque des revendications 4 à 6, dans lequel l'hydrolyse est conduite à une température de 0 à 30°C.

8. Procédé suivant l'une quelconque des revendications précédentes, dans lequel l'anthocyanidine formée a la formule :

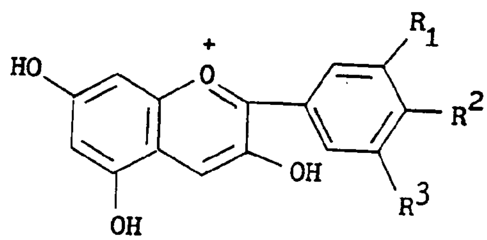

$X^-$

dans laquelle $R_1$, $R_2$ et $R_3$ sont indépendamment choisi parmi un atome d'hydrogène, un groupe hydroxy ou méthoxy et $X^-$ est un anion.

9. Procédé suivant la revendication 8, dans lequel $R_1$, $R_2$ et $R_3$ ont l'une des significations suivantes :
   - a $R_1$=$R_3$=H, $R_2$=OH
   - b $R_1$=$R_2$=OH, $R_3$=H
   - c $R_1$=$R_2$=$R_3$=OH
   - d $R_1$=OMe, $R_2$=OH, $R_3$=H
   - e $R_1$=$R_2$=OH, $R_3$=OMe
   - f $R_1$=$R_3$=OMe, $R_2$=OH

10. Composé, qui est le 2,4-di-O-benzoyl phloroglucinaldéhyde ayant la formule :

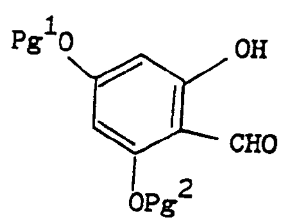

VII

sous une forme pratiquement pure, dans laquelle $Pg^1$ et $Pg^2$ représentent chacun un groupe benzoyle.

11. Utilisation du 2,4-di-O-benzoyl phloroglucinaldéhyde ayant la formule :

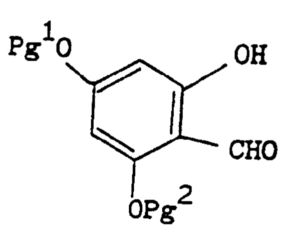

VII

dans laquelle $Pg^1$ et $Pg^2$ représentent chacun un groupe benzoyle, en tant qu'intermédiaire dans la synthèse d'anthocyanidines.